# FASCICULE DE BREVET EUROPEEN

(11) **EP 0 802 784 B1**
(45) Date de publication et mention de la délivrance du brevet: **13.12.2000**
(21) Numéro de dépôt: 96901039.6
(22) Date de dépôt: 09.01.1996
(51) Int. Cl.: A61K 7/48

(54) **MILIEU NUTRITIF UTILISABLE COMME MILIEU DE CULTURE DE CELLULES EPIDERMIQUES ET APPLICATIONS**
NÄHRSTOFF FÜR EPIDERMALE ZELLKULTUR UND VERWENDUNG
NUTRIENT MEDIUM FOR USE AS A CULTURE MEDIUM FOR EPIDERMAL CELLS, AND USES THEREOF

(30) Priorité: 09.01.1995 FR 9500327; 09.01.1995 FR 9500329
(43) Date de publication de la demande: 29.10.1997
(73) Titulaire: Thorel, Jean-Noel, F-75014 Paris (FR)
(72) Inventeur: THOREL, Jean-No[l, F-75014 Paris (FR); GATTO, Hugues, F-73200 Albertville (FR)
(74) Mandataire: Guerre, Dominique
(86) Numéro de dépôt international: FR9600037
(87) Numéro de publication internationale: WO9621421

(56) Documents cités:
- WO-A-94/13260
- FR-A- 2 694 692
- PATENT ABSTRACTS OF JAPAN vol. 11, no. 193 (C-430) [2640] , 20 Juin 1987 & JP,A,62 019511 (KANEBO LTD.), 28 Janvier 1987,

## Description

La présente invention concerne un milieu nutritif complexe, ses applications et plus particulièrement son utilisation pour fabriquer une composition à usage topique, et notamment cosmétique ou médicamenteuse.

On connaît selon le document WO-A-94/13260 une composition cosmétique ou dermatologique destinée à traiter divers troubles de la kératinisation ou de la pigmentation de l'épiderme, comprenant un extrait de Simarouba qui intervient en améliorant la différenciation des kératinocytes. Ce document décrit aussi l'utilisation de l'extrait de Simarouba pour préparer un milieu de culture des kératinocytes, dans lequel il se trouve en association avec un milieu de base nutritif (milieu MDEM), un facteur de croissance et du sérum de veau foetal, car l'extrait ne permet pas à lui-seul aux kératinocytes de proliférer.

Selon l'invention on apporte un milieu dont la composition permet de créer un environnement extra-cellulaire parfaitement adapté à l'épiderme, en fournissant en particulier :
- un apport nutritionnel optimisé, aussi bien en vitamines, oligo-éléments, qu'en acides aminés essentiels,
- des facteurs de croissance cellulaire, visant à substituer les interactions cellulaires morphogènes,
- et des caractéristiques de pH et d'osmolarité proches des conditions physiologiques.

De manière générale, conformément à l'invention l'agent nutritionnel consiste en un milieu nutritif complexe, comprenant des composés à la fois biocompatibles, biomimétiques et biodisponibles au niveau cutané, à l'exclusion de tout extrait biologique d'origine animale, tel que sérum de veau foetal, ou d'origine cellulaire.

Le milieu nutritif complexe retenu selon l'invention a une composition qui consiste en au moins des acides aminés, une vitamine, un facteur de croissance cellulaire, et un sel minéral, excluant tout extrait biologique d'origine animale ou d'origine cellulaire, et qui est adaptée pour permettre audit milieu seul et en milieu aqueux, une culture in vitro viable d'un inoculum de kératinocytes épidermiques humains avec au moins une prolifération clonale de ces derniers au premier passage, sans assise nourricière vivante telle que les fibroblastes.

Par "biocompatible", on entend la propriété selon laquelle le composé présente une innocuité au niveau cutané.

Par "biomimétique", on entend le fait que le composé est présent à l'état naturel dans la peau.

Par "biodisponible", on entend la propriété selon laquelle le composé est assimilable par les kératinocytes épidermiques humains, aussi bien in vitro qu'in vivo.

Par des essais de routine, l'homme de métier est à même de formuler un milieu nutritif complexe selon l'invention, en procédant en particulier avec ledit milieu à des cultures in vitro de kératinocytes, dont la croissance peut être observée, par exemple au microscope.

A cet égard, les documents suivants ont déjà décrit des milieux adaptés à des cultures in vitro de kératinocytes, dont la viabilité et la croissance peuvent être objectivées par les tests actuellement en vigueur, et être directement appréciées par observation sous microscope :
- Boyce ST, Ham RG, Calcium-regulated differentiation of normal human epidermal keratinocytes in defined clonal culture and serum-free serial culture, J. Invest. Dermatol. 1983; 81: 33S-40S
- Boyce ST, Ham RG, Cultivation, frozen storage, and clonal growth of normal human epidermal keratinocytesin serum-free media, J. Tissue Culture Methods. 1985; 9: 83-93.

En tant que de besoin, le contenu de ces publications est incorporé à la présente description.

Le milieu nutritif complexe selon l'invention comprend des acides aminés, une ou plusieurs vitamines, un ou plusieurs facteurs de croissance cellulaire, et un ou plusieurs sels minéraux.

Une composition à usage topique de l'invention comprend une phase biocompatible avec les parties superficielles du corps humain, dans laquelle est distribué de manière homogène au moins ledit milieu nutritif tel que défini ci-dessus.

Dans une composition selon la présente invention, la phase biocompatible dans laquelle est distribué l'agent nutritionnel peut constituer l'excipient, ou l'un des composants de l'excipient de ladite composition.

L'ensemble des composés présents dans le milieu nutritif selon l'invention étant hydrosolubles, deux voies de formulation peuvent être mises en oeuvre pour obtenir une composition à usage topique :
**1)** Phase continue aqueuse, contenant le milieu nutritif selon l'invention :
   - sous forme de gel aqueux, à l'aide d'un polymère hydrosoluble non ionique du type polysaccharide ou éther de cellulose (polymères compatibles avec la forte charge ionique du milieu);
   - sous forme de système émulsionné (émulsion d'huile dans l'eau faisant appel à des tensio-actifs résistant aux fortes charges ioniques);
   - sous forme de sérum cosmétique.
**2)** Phase continue huileuse, la phase discontinue contenant le milieu nutritif selon l'invention :
   - sous forme émulsionnée, étant entendu que la force ionique de la phase discontinue implique l'instabilité de l'émulsion ; il est cependant possible de formuler des phases lamellaires ou cylindriques présentant une meilleure stabilité, ou encore un système bi-phasique remis extemporanément en émulsion par simple agitation;
   - par encapsulation :
      * dans une capsule rigide, du type polysaccharide, dispersée dans la phase lipidique,
      * dans une capsule molle, du type gélatine, dispersée dans la phase discontinue.

L'utilisation de liposomes connue vecteur d'encapsulation est envisageable sous forme d'un gel liposomal en phase continue aqueuse.

Une composition selon l'invention peut servir de base cosmétique. Son apport nutritionnel est notablement intéressant pour l'amélioration de la viabilité, le maintien de l'intégrité et l'équilibre des cellules cutanées superficielles. En particulier elle permet de préserver durablement les qualités intrinsèques primaires de la peau, d'augmenter sa résistance aux agressions et de favoriser, le cas échéant, son retour à un état d'équilibre.

Un autre objet de l'invention est une préparation cosmétique comprenant une base précédemment définie, dans laquelle le milieu nutritif complexe constitue soit un principe actif, soit un excipient en présence d'autres principes actifs qu'il est susceptible de potentialiser.

Le milieu nutritif complexe de l'invention peut aussi être utilisé pour la préparation ou l'obtention d'un médicament.

L'utilisation d'un tel milieu sur une peau fragilisée (peaux irritées, desséchées, peaux sénescentes,...), permet de retrouver un état cutané satisfaisant tant en terme de trophicité que d'hydratation des couches superficielles de l'épiderme.

Une composition médicamenteuse comprenant un milieu nutritif complexe selon l'invention peut servir de base galénique, notamment nutritive.

Elle possède en outre des propriétés pharmacologiques qui seront mises en évidence dans les exemples. Selon une application avantageuse d'une composition médicamenteuse de l'invention, elle est destinée au traitement conservateur des greffons après leur prélèvement. Elle se présentera de manière préférentielle sous la forme d'une solution stérile particulièrement adaptée pour le nettoyage et l'entretien des greffons chez les grands brûlés.

En outre une composition telle que précédemment définie présente des propriétés performantes pour prévenir ou traiter des troubles de la cicatrisation tels que escarres, ulcères variqueux, vergetures, chéloïdes, et/ou un retard de la cicatrisation.

De manière plus générale, une composition selon l'invention peut être incorporée dans toute préparation à usage galénique, en tant que principe actif avec éventuellement d'autres principes actifs, mais également comme excipient grâce à sa capacité à potentialiser l'action de principes actifs spécifiques.

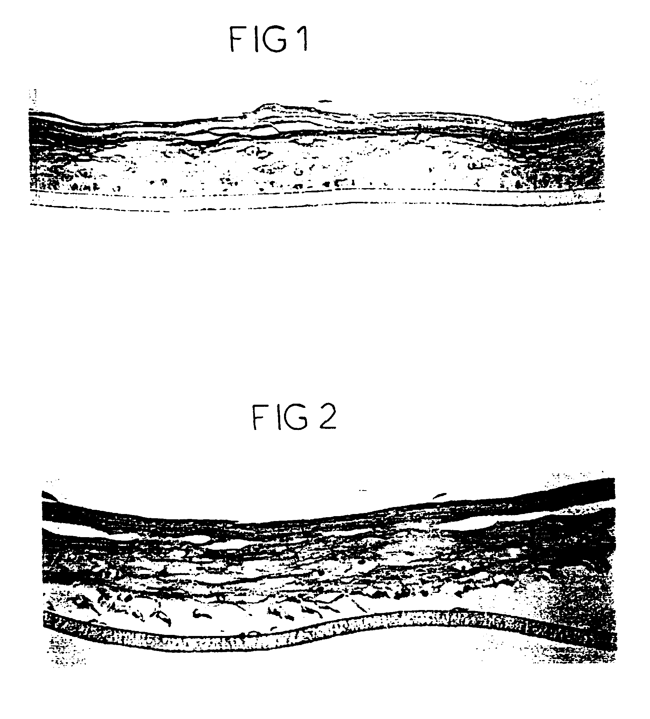

Les caractéristiques, applications et avantages de la présente invention sont exposés plus en détails dans les Exemples 1 à 4 et Figures 1 à 4 suivants.

L'Exemple 1 donne un exemple de formulation d'une composition de l'invention.

L'Exemple 2 met en évidence les propriétés d'une composition de l'invention par rapport à des milieux connus, à l'appui du dessin annexé dans lequel :
Fig. 1 est une vue en coupe d'épidermes humains après 36 heures de culture dans un milieu commercial standard dénommé MCDB 153, commercialisé notamment par IRVINE SCIENTIFIC et GIBCO-BRL,
Fig. 2 est une vue en coupe d'épidermes humains après 36 heures de culture dans une solution saline tamponnée (PBS), solution saline équilibrée couramment utilisée en culture cellulaire, et
Fig. 3 est une vue en coupe d'épidermes humains en culture dans le milieu nutritif de l'invention, décrit à l'Exemple 1 à différents temps de culture :
   A : au bout de 12 heures
   B : au bout de 24 heures
   C : au bout de 36 heures

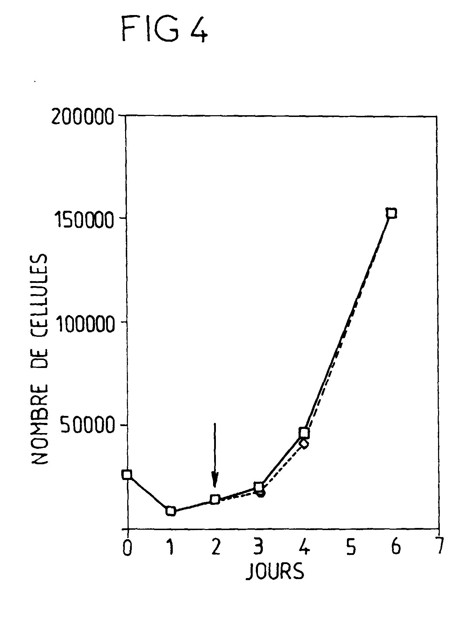

L'Exemple 3 met en évidence l'absence de stimulation de la prolifération de cellules transformées par une composition de l'invention par rapport à une composition standard, à l'appui de la Fig.4 qui représente un diagramme montrant la multiplication de cellules transformées cultivées sur un milieu de l'invention et un milieu standard.

L'Exemple 4 illustre les propriétés pharmacologiques d'une composition de l'invention : a) sur le traitement de greffons ; b) sur la cicatrisation.

### Exemple 1:

### Formulation d'une composition de l'invention

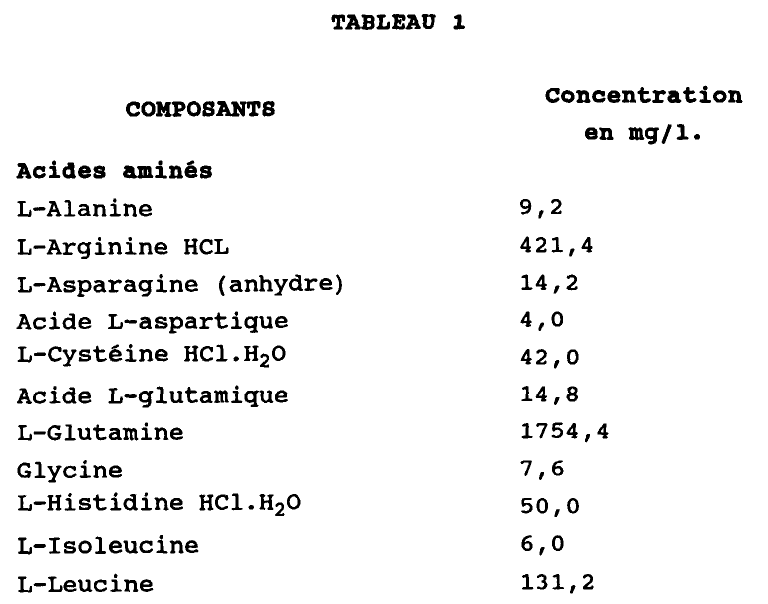

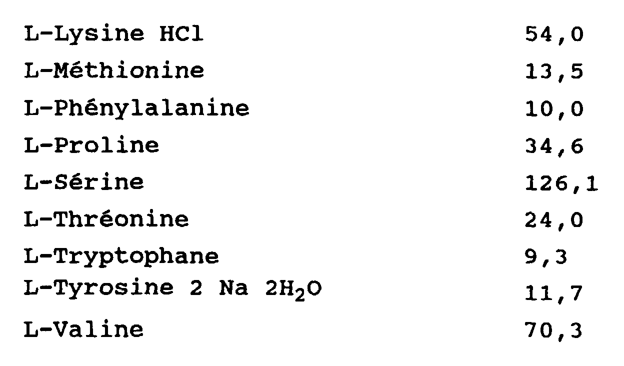

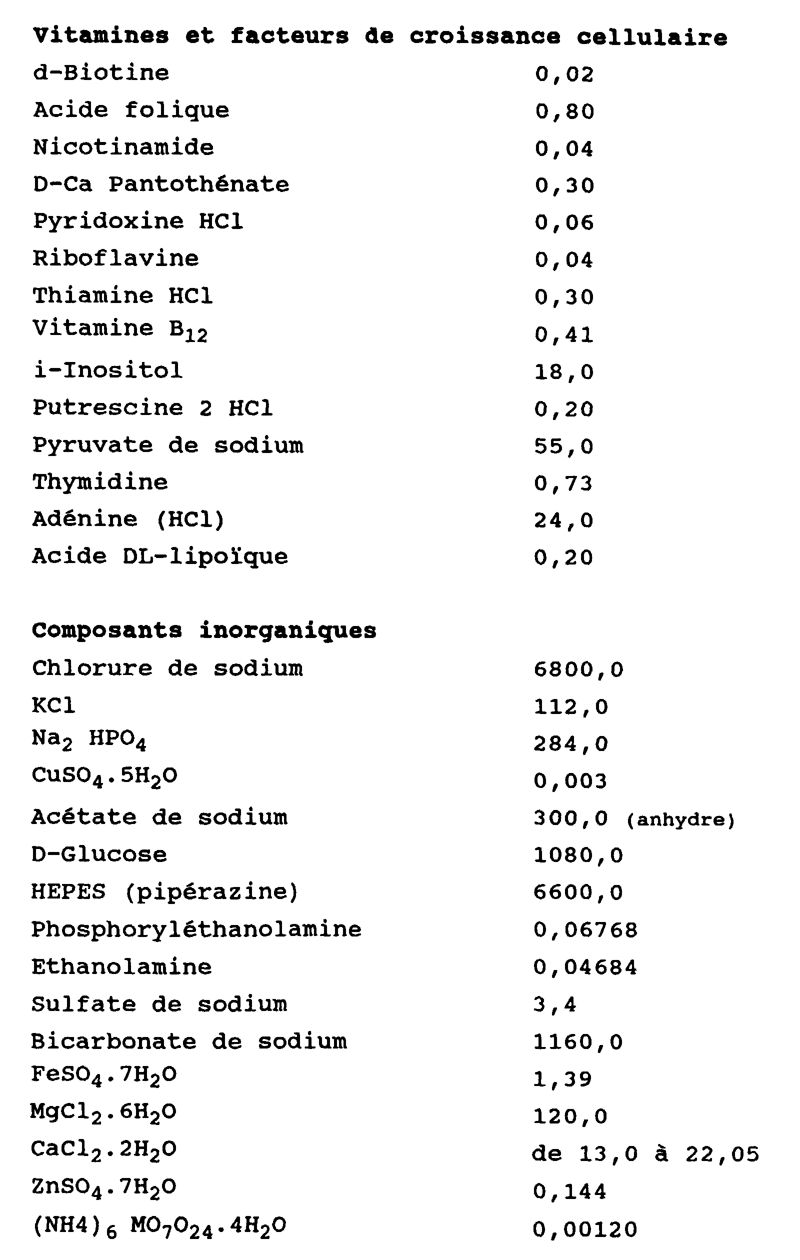

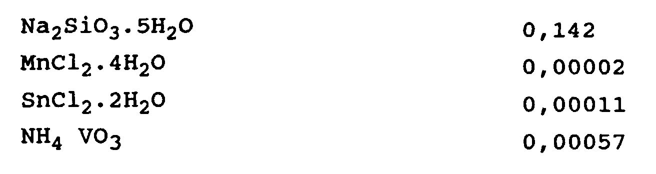

### Exemple 2:

La cytocompatibilité et les performances du milieu nutritif complexe décrit à l'Exemple 1 ont été testées sur des cultures de kératinocytes humains en monocouche, et sur des épidermes humains reconstitués in vitro.

Le milieu nutritif selon l'Exemple 1 permet la culture de kératinocytes en monocouche dans des conditions optimales de viabilité, durant au moins 36 heures, sans que ne se manifeste le moindre effet cytotoxique.

A l'inverse, une solution de survie classique telle que le PBS (Phosphate Buffered Saline, solution saline équilibrée couramment utilisée en culture cellulaire) s'avère cytotoxique dès 12 heures d'incubation.

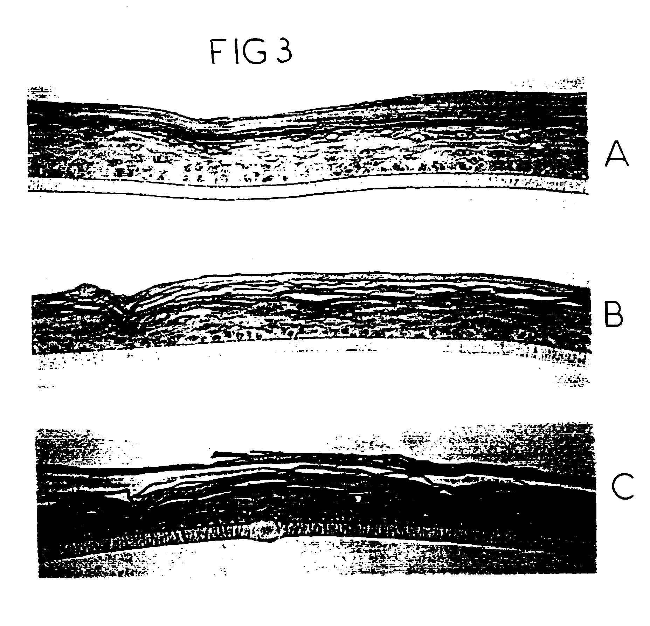

Conformément à la Fig 3, Le milieu nutritif selon l'exemple autorise une culture d'épidermes humains normaux reconstitués dans des conditions optimales de viabilité, sans manifestations cytotoxiques même après 36 heures (Fig 3C) de mise en contact. Les cultures présentaient des couches cellulaires basales, spineuses, granuleuses et cornées intactes, orthokératosiques, de stratification régulière et normale.

En comparant la Fig 3C avec la Fig 1, cette dernière illustrant l'utilisation d'un milieu commercial standard (MCDB 153, commercialisé notamment par IRVINE SCIENTIFIC), on voit que les performances du milieu de l'invention sont aussi bonnes.

Par contre, l'utilisation de PBS induit, conformément à Fig 2, l'apparition de kératinocytes en phase terminale de différenciation au niveau des assises basales et spineuses, avec des signes de nécrose plus ou moins prononcés. On note également un décollement total de l'épiderme, avec destructuration complète des différentes assises kératinocytaires.

**Exemple 3:** Effets d'une composition de l'invention sur la croissance de cellules épidermiques transformées.

La composition utilisée pour cette étude est celle décrite à l'Exemple 1 comprenant le milieu dit milieu 1.

L'effet de la composition 1 sur la croissance d'une lignée de kératinocytes humains spontanément transformée a été testé durant 4 jours de culture par comparaison avec des cellules cultivées sur un milieu standard (DMEM, Dulbeco Modified Epidermal Medium + sérum de veau foetal).

Les cellules sont premièrement ensemencées dans le milieu standard et poussent jusqu'au 2e jour après ensemencement dans ce milieu. Au 2e jour, le lot de cellules est partagé en deux, un lot continuant à être cultivé en milieu standard, l'autre en milieu 1.

Les résultats sont rassemblés sur la figure 4 dans laquelle la courbe obtenue avec les points ― 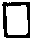 ― correspond à la composition de l'invention, et celle obtenue avec les points --□-- correspond à la composition de milieu standard. Les points ont été doublés et les comptages proviennent de quadruplicate. Les résultats sont corrigés de l'écart standard à la moyenne, SEM. La flèche apparaissant sur le diagramme correspond au partage du lot au second jour de culture.

La morphologie des cellules est différente selon le milieu employé. Celle des cellules cultivées en milieu 1 se rapproche davantage de celle obtenue en utilisant un milieu semi-défini pour cellules épithéliales, type KSFM de GIBCO-BRL (cellules aux jonctions plus lâches, aspect moins pavimenteux...).

Il n'est pas noté de différence significative dans la pousse de cette lignée en fonction des différents milieux, jusqu'à confluence (jours 6 à 7, non montré ici).

On conclut que la composition 1 n'a aucun effet stimulant sur la prolifération des kératinocytes transformés.

**Exemple 4:** Effets d'une composition de l'invention sur la prise de greffes de peaux humaines et la prévention des troubles de cicatrisation.

La composition testée est celle décrite à l'Exemple 1 comprenant le milieu dit milieu 1.

Les effets de la composition 1 sur la prise de greffes de peaux humaines et la prévention des troubles de la cicatrisation ont été étudiés sur un modèle murin (souris athymique dépourvue d'immunité à médiation cellulaire).

Deux types de greffons ont été employés: épidermes de culture et peaux humaines issues de chirurgie plastique. Les greffons ont été irrigués durant 30 jours avec 1 ml de composition 1 (une application par jour) pour les souris du groupe A et 1 ml de solution saline tamponnée (PBS) pour les souris du groupe B (20 animaux par groupe). Des compresses de tulle gras ont été appliquées après chaque irrigation afin d'éviter la dessication des greffons.

Une observation clinique des greffes a été réalisée à J-7, J-15 et J-30.

Deux paramètres ont été évalués : la nécrose des épidermes de culture et la cicatrisation.

### a) la nécrose des épidermes de culture ("prise de greffes")

La cotation est effectuée de 0 à 3 : 0= aucun signe de nécrose; 1= inflammation légère et altération superficielle du greffon; 2= nécrose partielle; 3= nécrose totale.

Les résultats sont rassemblés dans le Tableau 2.

**TABLEAU 2**

| **SOURIS DU GROUPE A** (20 greffons au total traités par la composition nutritive) | | | |
|---|---|---|---|
| **Cotations** | J-7 | J-15 | J-30 |
| 0 | 9/20 | 12/20 | 16/20 |
| 1 | 7/20 | 4/20 | 0/20 |
| 2 | 3/20 | 2/20 | 2/20 |
| 3 | 1/20 | 2/20 | 2/20 |

| **SOURIS DU GROUPE B** (20 greffons traités par la solution saline tamponée) | | | |
|---|---|---|---|
| **Cotations** | J-7 | J-15 | J-30 |
| 0 | 2/20 | 4/20 | 7/20 |
| 1 | 8/20 | 6/20 | 3/20 |
| 2 | 6/20 | 5/20 | 5/20 |
| 3 | 4/20 | 5/20 | 5/20 |

La composition 1 améliore la prise des greffes d'épidermes humains de culture sur souris athymiques par rapport à une solution de survie classique (PBS). Des différences significatives sont notées dès 7 jours de traitement pour une amélioration finale de plus de 50%.

### b) la cicatrisation (avec les peaux totales greffées)

Une cotation est effectuée de 0 à 3 : 0 = aucun trouble de cicatrisation; 1= retard de cicatrisation ; 2 = retard avec anomalie de la cicatrisation (bourgeonnement de la cicatrice) ; 3 = cicatrice hypertrophique.

Les résultats sont rassemblés dans le Tableau 3.

**TABLEAU 3**

| **SOURIS DU GROUPE A** (20 peaux totales greffées et traitées par la composition nutritive) | | | |
|---|---|---|---|
| **Cotations** | J-7 | J-15 | J-30 |
| 0 | 20/20 | 16/20 | 15/20 |
| 1 | 0/20 | 3/20 | 2/20 |
| 2 | 0/20 | 1/20 | 2/20 |
| 3 | 0/20 | 0/20 | 1/20 |

| **SOURIS DU GROUPE B** (20 peaux totales greffées et traitées par la solution saline tamponnée) | | | |
|---|---|---|---|
| **Cotations** | J-7 | J-15 | J-30 |
| 0 | 16/20 | 10/20 | 5/20 |
| 1 | 4/20 | 7/20 | 8/20 |
| 2 | 0/20 | 3/20 | 3/20 |
| 3 | 0/20 | 0/20 | 4/20 |

La composition 1 améliore de façon significative les processus de cicatrisation; cet effet est particulièrement marqué après 30 jours de traitement.

## Revendications

1. Utilisation d'un milieu nutritif complexe pour la fabrication ou l'obtention d'une composition à usage topique, ledit milieu nutritif complexe consistant en au moins des acides aminés, une vitamine, un facteur de croissance cellulaire, et un sel minéral, et excluant tout extrait biologique d'origine animale ou d'origine cellulaire, ledit milieu permettant à lui seul et en milieu aqueux, une culture viable in vitro d'un inoculum de kératinocytes épidermiques humains, avec au moins une prolifération clonale de ces derniers au premier passage, sans assise nourricière vivante.

2. Utilisation selon la revendication 1, caractérisée en ce que les composants du milieu nutritif sont à la fois biocompatibles, bio-mimétiques et biodisponibles au niveau cutané.

3. Utilisation selon la revendication 1, caractérisée en ce que le milieu nutritif complexe a la composition suivante, la concentration des composants étant exprimée en mg/l :
| **Acides aminés** | |
|---|---|
| L-Alanine | 9,2 |
| L-Arginine HCL | 421,4 |
| L-Asparagine (anhydre) | 14,2 |
| Acide L-aspartique | 4,0 |
| L-Cystéine HCL. H₂O | 42,0 |
| Acide L-Glutamique | 14,8 |
| L-Glutamine | 1754,4 |
| Glycine | 7,6 |
| L-Histidine HCL. H₂O | 50,0 |
| L-Isoleucine | 6,0 |
| L-Leucine | 131,2 |
| L-Lysine HCl | 54,0 |
| L-Méthionine | 13,5 |
| L-Phénylalanine | 10,0 |
| L-Proline | 34,6 |
| L-Sérine | 126,1 |
| L-Thréonine | 24,0 |
| L-Tryptophane | 9,3 |
| L-Tyrosine 2 Na 2H₂O | 11,7 |
| L-Valine | 70,3 |
| **Vitamines et facteurs de croissance cellulaire** | |
|---|---|
| d-Biotine | 0,02 |
| Acide folique | 0,80 |
| Nicotinamide | 0,04 |
| D-Ca Pantothénate | 0,30 |
| Pyridoxine HCl | 0,06 |
| Riboflavine | 0,04 |
| Thiamine HCl | 0,30 |
| Vitamine B₁₂ | 0,41 |
| i-Inositoi | 18,0 |
| Putrescine 2 HCl | 0,20 |
| Pyruvate de sodium | 55,0 |
| Thymidine | 0,73 |
| Adénine (HCl) | 24,0 |
| Acide DL-lipoïque | 0,20 |
| **Composants inorganiques** | |
|---|---|
| Chlorure de sodium | 6800,0 |
| KCl | 112,0 |
| Na₂ HPO₄ | 284,0 |
| CuSO₄.5H₂O | 0,003 |
| Acétate de sodium | 300,0 (anhydre) |
| D-Glucose | 1080,0 |
| HEPES (pipérazine) | 6600,0 |
| Phosphoryléthanolamine | 0,06768 |
| Ethanolamine | 0,04684 |
| Sulfate de sodium | 3,4 |
| Bicarbonate de sodium | 1160,0 |
| FeSO₄.7H₂O | 1,39 |
| MgCl₂.6H₂O | 120,0 |
| CaCl₂.2H₂O | de 13,0 à 22,05 |
| ZnSO₄.7H₂O | 0,144 |
| (NH₄)₆ MO₇O₂₄.4H₂O | 0,00120 |
| Na₂SiO₃.5H₂O | 0,142 |
| MnCl₂.4H₂O | 0,00002 |
| SnCl₂.2H₂O | 0,00011 |
| NH₄ VO₃ | 0,00057 |

4. Composition à usage topique comprenant une phase biocompatible avec les parties superficielles du corps humain, dans laquelle est distribué de manière homogène au moins un milieu nutritif tel que défini selon l'une quelconque des revendications 1 à 3.

5. Composition selon la revendication 4, caractérisée en ce qu'elle se présente sous forme biphasique, avec une phase continue aqueuse contenant le milieu nutritif complexe, et notamment sous forme de gel aqueux, ou d'émulsion d'huile dans l'eau.

6. Composition selon la revendication 4, caractérisée en ce qu'elle se présente sous forme biphasique, avec une phase continue huileuse, notamment sous forme d'émulsion, la phase discontinue contenant le milieu nutritif complexe.

7. Base cosmétique comprenant une composition selon l'une quelconque des revendications 4 à 6.

8. Préparation cosmétique comprenant une base cosmétique selon la revendication 7, caractérisée en ce que le milieu nutritif complexe constitue soit un principe actif, soit un excipient, notamment potentialisant un autre principe actif.

9. Utilisation d'un milieu nutritif complexe consistant en au moins des acides aminés, une vitamine, un facteur de croissance cellulaire, et un sel minéral, et excluant tout extrait biologique d'origine animale ou d'origine cellulaire, ledit milieu étant adapté pour permettre à lui seul et en milieu aqueux, une culture viable in vitro d'un inoculum de kératinocytes épidermiques humains, avec au moins une prolifération clonale de ces derniers au premier passage, sans assise nourricière vivante, pour la fabrication ou obtention d'un médicament.

10. Utilisation selon la revendication 9, caractérisée en ce que les composés du milieu nutritif sont à la fois biocompatibles, bio-mimétiques et biodisponibles au niveau cutané.

11. Utilisation selon la revendication 9, caractérisée en ce que le milieu nutritif complexe a la composition suivante, la concentration des composants étant exprimée en mg/l :
| **Acides aminés** | |
|---|---|
| L-Alanine | 9,2 |
| L-Arginine HCl | 421,4 |
| L-Asparagine (anhydre) | 14,2 |
| Acide L-aspartique | 4,0 |
| L-Cystéine HCl.H₂O | 42,0 |
| Acide L-Glutamique | 14,8 |
| L-Glutamine | 1754,4 |
| Glycine | 7,6 |
| L-Histidine HCl.H₂O | 50,0 |
| L-Isoleucine | 6,0 |
| L-Leucine | 131,2 |
| L-Lysine HCl | 54,0 |
| L-Méthionine | 13,5 |
| L-Phénylalanine | 10,0 |
| L-Proline | 34,6 |
| L-Sérine | 126,1 |
| L-Thréonine | 24,0 |
| L-Tryptophane | 9,3 |
| L-Tyrosine 2 Na 2H₂O | 11,7 |
| L-Valine | 70,3 |
| **Vitamines et facteurs de croissance cellulaire** | |
|---|---|
| d-Biotine | 0,02 |
| Acide folique | 0,80 |
| Nicotinamide | 0,04 |
| D-Ca Pantothénate | 0,30 |
| Pyridoxine HCl | 0,06 |
| Riboflavine | 0,04 |
| Thiamine HCl | 0,30 |
| Vitamine B₁₂ | 0,41 |
| i-Inositol | 18,0 |
| Putrescine 2 HCl | 0,20 |
| Pyruvate de sodium | 55,0 |
| Thymidine | 0,73 |
| Adénine (HCl) | 24,0 |
| Acide DL-lipoïque | 0,20 |
| **Composants inorganiques** | |
|---|---|
| Chlorure de sodium | 6800,0 |
| KCl | 112,0 |
| Na₂ HPO₄ | 284,0 |
| CuSO₄.5H₂O | 0,003 |
| Acétate de sodium | 300,0 (anhydre) |
| D-Glucose | 1080,0 |
| HEPES (pipérazine) | 6600,0 |
| Phosphoryléthanolamine | 0,06768 |
| Ethanolamine | 0,04684 |
| Sulfate de sodium | 3,4 |
| Bicarbonate de sodium | 1160,0 |
| FeSO₄.7H₂O | 1,39 |
| MgCl₂.6H₂O | 120,0 |
| CaCl₂.2H₂O | de 13,0 à 22,05 |
| ZnSO₄.7H₂O | 0,144 |
| (NH₄)₆ MO₇O₂₄.4H₂O | 0,00120 |
| Na₂SiO₃.5H₂O | 0,142 |
| MnCl₂.4H₂O | 0,00002 |
| SnCl₂.2H₂O | 0,00011 |
| NH₄ VO₃ | 0,00057 |

12. Utilisation selon l'une quelconque des revendications 9 à 11, caractérisée en ce que l'agent nutritionnel constitue l'un des, sinon le principe actif dudit médicament.

13. Utilisation selon l'une quelconque des revendications 9 à 12 pour obtenir un médicament destiné au traitement conservateur des greffons.

14. Utilisation selon l'une quelconque des revendications 9 à 13 pour prévenir ou traiter les troubles et/ou retard de la cicatrisation.

15. Composition médicamenteuse à usage topique comprenant une phase biocompatible avec les parties superficielles du corps humain, dans laquelle est distribué de manière homogène au moins un milieu nutritif tel que défini selon l'une quelconque des revendications 9 à 11.

16. Composition selon la revendication 15, caractérisée en ce qu'elle se présente sous forme biphasique, avec une phase continue aqueuse contenant le milieu nutritif complexe, et notamment sous forme de gel aqueux, ou d'émulsion d'huile dans l'eau.

17. Composition selon la revendication 15, caractérisée en ce qu'elle se présente sous forme biphasique, avec une phase continue huileuse, notamment sous forme d'émulsion, la phase discontinue contenant le milieu nutritif complexe.

18. Base galénique comprenant une composition selon l'une quelconque des revendications 15 à 17.

19. Base galénique selon la revendication 18, caractérisée en ce qu'elle est destinée au traitement conservateur des greffons.

20. Base galénique selon la revendication 18, caractérisée en ce qu'elle est destinée à la prévention ou au traitement des troubles et/ou retard de la cicatrisation.

## Claims

1. Use of a complex nutrient medium for the manufacture or production of a composition for topical use, the said complex nutrient medium consisting of at least some amino acids, a vitamin, a cell growth factor and an inorganic salt, and excluding any biological extract of animal origin or of cellular origin, the said medium permitting, on its own and in an aqueous medium, viable in vitro culture of an inoculum of human epidermal keratinocytes, with at least one clonal proliferation of the latter at the first passage, without a living nourishing substrate.

2. Use according to Claim 1, characterized in that the components of the nutrient medium are both biocompatible, biomimetic and bioavailable in respect of the skin.

3. Use according to Claim 1, characterized in that the complex nutrient medium has the following composition, the concentration of the components being expressed in mg/l:
| **Amino acids** | |
|---|---|
| L-Alanine | 9.2 |
| L-Arginine HCl | 421.4 |
| L-Asparagine (anhydrous) | 14.2 |
| L-Aspartic acid | 4.0 |
| L-Cysteine HCl. H₂O | 42.0 |
| L-Glutamic acid | 14.8 |
| L-Glutamine | 1754.4 |
| Glycine | 7.6 |
| L-Histidine HCl. H₂O | 50.0 |
| L-Isoleucine | 6.0 |
| L-Leucine | 131.2 |
| L-Lysine HCl | 54.0 |
| L-Methionine | 13.5 |
| L-Phenylalanine | 10.0 |
| L-Proline | 34.6 |
| L-Serine | 126.1 |
| L-Threonine | 24.0 |
| L-Tryptophan | 9.3 |
| L-Tyrosine 2 Na 2H₂O | 11.7 |
| L-Valine | 70.3 |
| **Vitamins and cell growth factors** | |
|---|---|
| d-Biotin | 0.02 |
| Folic acid | 0.80 |
| Nicotinamide | 0.04 |
| Ca D-Pantothenate | 0.30 |
| Pyridoxine HCl | 0.06 |
| Riboflavin | 0.04 |
| Thiamine HCl | 0.30 |
| Vitamin B₁₂ | 0.41 |
| i-Inositol | 18.0 |
| Putrescine 2 HCl | 0.20 |
| Sodium pyruvate | 55.0 |
| Thymidine | 0.73 |
| Adenine (HCl) | 24.0 |
| DL-Lipoic acid | 0.20 |
| **Inorganic components** | |
|---|---|
| Sodium chloride | 6800.0 |
| KCl | 112.0 |
| Na₂ HPO₄ | 284.0 |
| CuSO₄.5H₂O | 0.003 |
| Sodium acetate | 300.0 (anhydrous) |
| D-Glucose | 1080.0 |
| HEPES (piperazine) | 6600.0 |
| Phosphorylethanolamine | 0.06768 |
| Ethanolamine | 0.04684 |
| Sodium sulphate | 3.4 |
| Sodium bicarbonate | 1160.0 |
| FeSO₄.7H₂O | 1.39 |
| MgCl₂.6H₂O | 120.0 |
| CaCl₂.2H₂O | from 13.0 to 22.05 |
| ZnSO₄.7H₂O | 0.144 |
| (NH4)₆ MO₇O₂₄.4H₂O | 0.00120 |
| Na₂SiO₃.5H₂O | 0.142 |
| MnCl₂.4H₂O | 0.00002 |
| SnCl₂.2H₂O | 0.00011 |
| NH₄ VO₃ | 0.00057 |

4. Composition for topical use comprising a phase which is biocompatible with the superficial parts of the human body, in which phase at least one nutrient medium as defined according to any one of Claims 1 to 3 is distributed homogeneously.

5. Composition according to Claim 4, characterized in that it is in two-phase form, with an aqueous continuous phase containing the complex nutrient medium, and in particular in the form of an aqueous gel or an oil-in-water emulsion.

6. Composition according to Claim 4, characterized in that it is in two-phase form, with an oily continuous phase, in particular in emulsion form, the discontinuous phase containing the complex nutrient medium.

7. Cosmetic base comprising a composition according to any one of Claims 4 to 6.

8. Cosmetic preparation comprising a cosmetic base according to Claim 7, characterized in that the complex nutrient medium constitutes either an active principle, or an excipient, in particular one that potentiates another active principle.

9. Use of a complex nutrient medium consisting of at least one of the amino acids, a vitamin, a cell growth factor and an inorganic salt, and excluding any biological extract of animal origin or of cellular origin, the said medium being suitable for permitting, on its own and in an aqueous medium, viable in vitro culture of an inoculum of human epidermal keratinocytes, with at least one clonal proliferation of the latter at the first passage, without a living nourishing substrate, for the manufacture or production of a medicament.

10. Use according to Claim 9, characterized in that the components of the nutrient medium are both biocompatible, biomimetic and bioavailable in respect of the skin.

11. Use according to Claim 9, characterized in that the complex nutrient medium has the following composition, the concentration of the components being expressed in mg/l:
| **Amino acids** | |
|---|---|
| L-Alanine | 9.2 |
| L-Arginine HCl | 421.4 |
| L-Asparagine (anhydrous) | 14.2 |
| L-Aspartic acid | 4.0 |
| L-Cysteine HCl.H₂O | 42.0 |
| L-Glutamic acid | 14.8 |
| L-Glutamine | 1754.4 |
| Glycine | 7.6 |
| L-Histidine HCl.H₂O | 50.0 |
| L-Isoleucine | 6.0 |
| L-Leucine | 131.2 |
| L-Lysine HCl | 54.0 |
| L-Methionine | 13.5 |
| L-Phenylalanine | 10.0 |
| L-Proline | 34.6 |
| L-Serine | 126.1 |
| L-Threonine | 24.0 |
| L-Tryptophan | 9.3 |
| L-Tyrosine 2 Na 2H₂O | 11.7 |
| L-Valine | 70.3 |
| **Vitamins and cell growth factors** | |
|---|---|
| d-Biotin | 0.02 |
| Folic acid | 0.80 |
| Nicotinamide | 0.04 |
| Ca D-Pantothenate | 0.30 |
| Pyridoxine HCl | 0.06 |
| Riboflavin | 0.04 |
| Thiamine HCl | 0.30 |
| Vitamin B₁₂ | 0.41 |
| i-Inositol | 18.0 |
| Putrescine 2 HCl | 0.20 |
| Sodium pyruvate | 55.0 |
| Thymidine | 0.73 |
| Adenine (HCl) | 24.0 |
| DL-Lipoic acid | 0.20 |
| **Inorganic components** | |
|---|---|
| Sodium chloride | 6800.0 |
| KCl | 112.0 |
| Na₂ HPO₄ | 284.0 |
| CuSO₄.5H₂O | 0.003 |
| Sodium acetate | 300.0 (anhydrous) |
| D-Glucose | 1080.0 |
| HEPES (piperazine) | 6600.0 |
| Phosphorylethanolamine | 0.06768 |
| Ethanolamine | 0.04684 |
| Sodium sulphate | 3.4 |
| Sodium bicarbonate | 1160.0 |
| FeSO₄.7H₂O | 1.39 |
| MgCl₂.6H₂O | 120.0 |
| CaCl₂.2H₂O | from 13.0 to 22.05 |
| ZnSO₄.7H₂O | 0.144 |
| (NH4)₆ MO₇O₂₄.4H₂O | 0.00120 |
| Na₂SiO₃.5H₂O | 0.142 |
| MnCl₂.4H₂O | 0.00002 |
| SnCl₂.2H₂O | 0.00011 |
| NH₄ VO₃ | 0.00057 |

12. Use according to any one of Claims 9 to 11, characterized in that the nutritional agent constitutes one of the active principles, if not the active principle, of the said medicament.

13. Use according to any one of Claims 9 to 12, for obtaining a medicament intended for the preservative treatment of grafts.

14. Use according to any one of Claims 9 to 13, for preventing or treating disorders and/or delay of cicatrization.

15. Medicinal composition for topical use, comprising a phase which is biocompatible with the superficial parts of the human body, in which phase at least one nutrient medium as defined according to any one of Claims 9 to 11 is distributed homogeneously.

16. Compostion according to Claim 15, characterized in that it is in two-phase form, with an aqueous continuous phase containing the complex nutrient medium, and in particular in the form of an aqueous gel or an oil-in-water emulsion.

17. Composition according to Claim 15, characterized in that it is in two-phase form, with an oily continuous phase, in particular in emulsion form, the discontinuous phase containing the complex nutrient medium.

18. Pharmaceutical formulation base comprising a composition according to any one of Claims 15 to 17.

19. Pharmaceutical formulation base according to Claim 18, characterized in that it is intended for the preservative treatment of grafts.

20. Pharmaceutical formulation base according to Claim 18, characterized in that it is intended for the prevention or treatment of disorders and/or delay of cicatrization.

## Patentansprüche

1. Verwendung eines komplexen Nährmilieus zur Herstellung oder Erlangung einer Zusammensetzung zur topischen Anwendung, wobei das komplexe Nährmilieu zumindest aus Aminosäuren, einem Vitamin, einem zellulären Wachstumsfaktor und einem Mineralsalz besteht, und jeglichen biologischen Extrakt tierischen oder zellulären Ursprungs ausschließt, wobei das Milieu allein oder in wäßrigem Medium, ohne eine lebende Nährzellschicht, eine in vitro lebensfähige Kultivierung eines Inokulums humaner epidermaler Keratinozyten mit zumindest einer klonalen Proliferation der letzteren bei der ersten Passage ermöglicht.

2. Verwendung nach Anspruch 1, dadurch gekennzeichnet, daß die Komponenten des Nährmilieus im Hautbereich zugleich biokompatibel, biomimetisch und bioverfügbar sind.

3. Verwendung nach Anspruch 1, dadurch gekennzeichnet, daß das komplexe Nährmilieu die nachfolgende Zusammensetzung hat, wobei die Konzentration der Komponenten in mg/l ausgedrückt ist:
| **Aminosäuren** | |
|---|---|
| L-Alanin | 9,2 |
| L-Arginin-HCl | 421,4 |
| L-Aspargin (Anhydrid) | 14,2 |
| L-Asparaginsäure | 4,0 |
| L-Cystein-HCl · H₂O | 42,0 |
| L-Glutaminsäure | 14,8 |
| L-Glutamin | 1754,4 |
| Glycin | 7,6 |
| L-Histidin-HCl · H₂O | 50,0 |
| L-Isoleucin | 6,0 |
| L-Leucin | 131,2 |
| L-Lysin-HCl | 54,0 |
| L-Methionin | 13,5 |
| L-Phenylalanin | 10,0 |
| L-Prolin | 34,6 |
| L-Serin | 126,1 |
| L-Threonin | 24,0 |
| L-Tryptophan | 9,3 |
| L-Tyrosin 2 Na 2H₂O | 11,7 |
| L-Valin | 70,3 |
| **Vitamine und zelluläre Wachstumsfaktoren** | |
|---|---|
| d-Biotin | 0,02 |
| Folsäure | 0,80 |
| Nikotinamid | 0,04 |
| D-Ca-Pantothenat | 0,30 |
| Pyridoxin-HCl | 0,06 |
| Riboflavin | 0,04 |
| Thiamin-HCl | 0,30 |
| Vitamin B₁₂ | 0,41 |
| i-Inositol | 18,0 |
| Putrescin 2 HCl | 0,20 |
| Natrium-Pyruvat | 55,0 |
| Thymidin | 0,73 |
| Adenin | 24,0 |
| DL-Liponsäure | 0,20 |
| **Anorganische Komponenten** | |
|---|---|
| Natriumchlorid | 6800,0 |
| KCl | 112,0 |
| Na₂ HPO₄ | 284,0 |
| CuSO₄ · 5H₂O | 0,003 |
| Natriumacetat | 300 (anhydriert) |
| D-Glukose | 1080,0 |
| HEPES (Piperazin) | 6600,0 |
| Phosphorylethanolamin | 0,06768 |
| Ethanolamin | 0,04684 |
| Natriumsulfat | 3,4 |
| Natriumbicarbonat | 1160,0 |
| FeSO₄ · 7H₂O | 1,39 |
| MgCl₂ · 6H₂O | 120,0 |
| CaCl₂ · 2H₂O | von 13,0 bis 22,05 |
| ZnSO₄ · 7H₂O | 0,144 |
| (NH₄)₆ Mo₇O₂₄ · 4H₂O | 0,00120 |
| Na₂SiO₃ · 5H₂O | 0,142 |
| MnCl₂ · 4H₂O | 0,00002 |
| SnCl₂ · 2H₂O | 0,00011 |
| NH₄VO₃ | 0,00057 |

4. Zusammensetzung zur topischen Anwendung, die eine mit den Oberflächenbereichen des menschlichen Körpers biokompatible Phase aufweist, in der in homogener Art und Weise zumindest ein Nährmilieu nach einem der Ansprüche 1 bis 3 verteilt ist.

5. Zusammensetzung nach Anspruch 4, dadurch gekennzeichnet, daß diese in zweiphasiger Form mit einer stetigen wäßrigen Phase vorliegt, die das komplexe Nährmilieu enthält, und zwar insbesondere in Gestalt eines wäßrigen Gels oder einer Emulsion von Öl in Wasser.

6. Zusammensetzung nach Anspruch 4, dadurch gekennzeichnet, daß diese in zweiphasiger Form vorliegt, mit einer stetigen öligen Phase, insbesondere in Gestalt einer Emulsion, wobei die unstetige Phase das komplexe Nährmilieu enthält.

7. Kosmetische Grundlage, die eine Zusammensetzung nach einem der Ansprüche 4 bis 6 enthält.

8. Kosmetisches Präparat, das eine kosmetische Grundlage nach Anspruch 7 enthält, dadurch gekennzeichnet, daß das komplexe Nährmilieu entweder einen Wirkstoff oder einen insbesondere einen anderen Wirkstoff potenzierender Träger bildet.

9. Verwendung eines komplexen Nährmediums, zumindest bestehend aus Aminosäuren, einem Vitamin, einem zellulären Wachstumsfaktor und einem Mineralsalz, und ohne jeglichen biologischen Extrakt tierischen oder zellulären Ursprungs, wobei sich das besagte Milieu allein oder in wäßrigem Medium dazu eignet, ohne eine lebende Nährzellschicht, eine in vitro lebensfähige Kultivierung eines Inokulums humaner epidermaler Keratinozyten mit zumindest einer klonalen Proliferation der letzteren bei der ersten Passage zu ermöglichen, zur Herstellung oder Erlangung eines Medikamentes.

10. Verwendung nach Anspruch 9, dadurch gekennzeichnet, daß die Komponenten des Nährmilieus im Hautbereich zugleich biokompatibel, biomimetisch und bioverfügbar sind.

11. Verwendung nach Anspruch 9, dadurch gekennzeichnet, daß das komplexe Nährmilieu die nachfolgende Zusammensetzung hat, wobei die Konzentration der Komponenten in mg/l ausgedrückt ist:
| **Aminosäuren** | |
|---|---|
| L-Alanin | 9,2 |
| L-Arginin-HCl | 421,4 |
| L-Aspargin (Anhydrid) | 14,2 |
| L-Asparaginsäure | 4,0 |
| L-Cystein-HCl · H₂O | 42,0 |
| L-Glutaminsäure | 14,8 |
| L-Glutamin | 1754,4 |
| Glycin | 7,6 |
| L-Histidin-HCl · H₂O | 50,0 |
| L-Isoleucin | 6,0 |
| L-Leucin | 131,2 |
| L-Lysin-HCl | 54,0 |
| L-Methionin | 13,5 |
| L-Phenylalanin | 10,0 |
| L-Prolin | 34,6 |
| L-Serin | 126,1 |
| L-Threonin | 24,0 |
| L-Tryptophan | 9,3 |
| L-Tyrosin 2 Na 2H₂O | 11,7 |
| L-Valin | 70,3 |
| **Vitamine und zelluläre Wachstumsfaktoren** | |
|---|---|
| d-Biotin | 0,02 |
| Folsäure | 0,80 |
| Nikotinamid | 0,04 |
| D-Ca-Pantothenat | 0,30 |
| Pyridoxin-HCl | 0,06 |
| Riboflavin | 0,04 |
| Thiamin-HCl | 0,30 |
| Vitamin B₁₂ | 0,41 |
| i-Inositol | 18,0 |
| Putrescin 2 HCl | 0,20 |
| Natrium-Pyruvat | 55,0 |
| Thymidin | 0,73 |
| Adenin (HCl) | 24,0 |
| DL-Liponsäure | 0,20 |
| **Anorganische Komponenten** | |
|---|---|
| Natriumchlorid | 6800,0 |
| KCl | 112,0 |
| Na₂ HPO₄ | 284,0 |
| CuSO₄ · 5H₂O | 0,003 |
| Natriumacetat | 300 (anhydriert) |
| D-Glukose | 1080,0 |
| HEPES (Piperazin) | 6600,0 |
| Phosphorylethanolamin | 0,06768 |
| Ethanolamin | 0,04684 |
| Natriumsulfat | 3,4 |
| Natriumbicarbonat | 1160,0 |
| FeSO₄ · 7H₂O | 1,39 |
| MgCl₂ · 6H₂O | 120,0 |
| CaCl₂ · 2H₂O | von 13,0 bis 22,05 |
| ZnSO₄ · 7H₂O | 0,144 |
| (NH₄)₆ Mo₇O₂₄ · 4H₂O | 0,00120 |
| Na₂SiO₃ · 5H₂O | 0,142 |
| MnCl₂ · 4H₂O | 0,00002 |
| SnCl₂ · 2H₂O | 0,00011 |
| NH₄VO₃ | 0,00057 |

12. Verwendung nach einem der Ansprüche 9 bis 11, dadurch gekennzeichnet, daß der Nährstoff einen der Wirkstoffe des Medikaments andernfalls den eigentlichen Wirkstoff des Medikamentes bildet.

13. Verwendung nach einem der Ansprüche 9 bis 12 zum Erhalten eines Medikamentes, das zur konservierenden Behandlung von Transplantaten bestimmt ist.

14. Verwendung nach einem der Ansprüche 9 bis 13 zum Vorbeugen oder Behandeln von Störungen und/oder Verzögerung der Narbenbildung.

15. Medikamentöse Zusammensetzung zur topischen Anwendung, die eine mit den Oberflächenbereichen des menschlichen Körpers biokompatible Phase aufweist, in der in homogener Art und Weise zumindest ein Nährmilieu nach einem der Ansprüche 9 bis 11 verteilt ist.

16. Zusammensetzung nach Anspruch 15, dadurch gekennzeichnet, daß diese in zweiphasiger Form mit einer stetigen wäßrigen Phase vorliegt, die das komplexe Nährmilieu enthält, und zwar insbesondere in Gestalt eines wäßrigen Gels oder einer Emulsion von Öl in Wasser.

17. Zusammensetzung nach Anspruch 15, dadurch gekennzeichnet, daß diese in zweiphasiger Form vorliegt, mit einer stetigen öligen Phase, insbesondere in Gestalt einer Emulsion, wobei die unstetige Phase das komplexe Nährmilieu enthält.

18. Galenische Grundlage, die eine Zusammensetzung nach einem der Ansprüche 15 bis 17 enthält.

19. Galenische Grundlage nach Anspruch 18, dadurch gekennzeichnet, daß diese zur konservierenden Behandlung von Transplantaten bestimmt ist.

20. Galenische Grundlage nach Anspruch 18, dadurch gekennzeichnet, daß diese zur Vorbeugung oder zur Behandlung von Störungen und/oder Verzögerung der Narbenbildung bestimmt ist.
